# EUROPEAN PATENT SPECIFICATION

(11) **EP 3 108 902 B1**
(45) Date of publication and mention of the grant of the patent: **18.07.2018**
(21) Application number: 16163179.1
(22) Date of filing: 31.03.2016
(51) Int. Cl.: A61L 2/20, B01B 1/00

(54) **METHOD FOR STERILISING CONTAINERS**
VERFAHREN ZUR BEHÄLTNISSTERILISATION
PROCÉDÉE POUR STÉRILISER DES RÉCIPIENTS

(30) Priority: 24.06.2015 IT UB20151618
(43) Date of publication of application: 28.12.2016
(60) Divisional application: 18157787.5
(73) Proprietor: Gea Procomac S.p.A., 43038 Sala Baganza (PR) (IT)
(72) Inventor: ABELLI, Paolo, 43123 PARMA (IT)
(74) Representative: Dondi, Silvia

(56) References cited:
- EP-A2- 0 321 908
- EP-A2- 0 956 054
- WO-A2-2009/013226
- US-A- 5 173 259

## Description

The present invention relates to a method for sterilising containers made of polyethylene or high-density polyethylene.

The reference sector is the bottling of so-called "sensitive" food products, i.e. products that are particularly sensitive to bacteriological contamination and oxidation, such as, for example, isotonic drinks, juices, nectars, soft drinks, teas, milk-based drinks, coffee-based drinks, etc., for which it is fundamental to prevent any microbiological contamination throughout the packaging stages.

In this context, the attention is focused on the decontamination of the primary packaging with which the product comes into contact, whether it is a bottle (or its parison), a pouch or flexible container or a cardboard container ("brick").

In particular, the present invention relates to the aseptic packaging of drinks in bottles made of thermoplastic material (preferably PET) of which the parison is sterilised. The sterile parison is then blown under sterile conditions, so as to obtain a sterile bottle ready to be filled directly, without intermediate sterilisation or rinsing steps.

Decontamination techniques of the prior art for containers envisage the use of chemical agents (e.g. aqueous solutions containing peracetic acid or hydrogen peroxide), or the application of radiation or the use of heat.

Chemical agents commonly used are peracetic acid and hydrogen peroxide. Throughout the present description and appended claims, the term "hydrogen peroxide" refers to an aqueous solution of hydrogen peroxide, preferably 35%.

The application of hydrogen peroxide generally takes place according to two methods.

A first chemical sterilisation method for sterilising the walls of a container envisages the following steps:
- vaporising an aqueous solution containing hydrogen peroxide into the container;
- making the hydrogen peroxide vapours condense on the inside walls of the container (bringing the walls themselves to a temperature less than or equal to the condensation temperature of the peroxide vapours);
- making the condensate evaporate, e.g. with hot air, so as to "activate" the hydrogen peroxide previously condensed;
- drying the inside walls of the container.

A second chemical sterilisation method envisages keeping the hydrogen peroxide constantly in the vapour state. The hydrogen peroxide is vaporised and the vapours are sent onto the surface to be sterilised. Having such surface at a temperature greater than the hydrogen peroxide vapour temperature means condensation does not occur throughout the entire sterilisation period. In this case, given the temperature of the vapours, it is not necessary to activate the hydrogen peroxide.

Both methods envisage a step in which an aqueous solution of hydrogen peroxide is brought from the liquid phase to the gaseous phase, known as VHP (Vapour Hydrogen Peroxide).

To generate VHP it is known that the liquid hydrogen peroxide can be dripped onto a hot plate (for example heated with electrical resistances) having a temperature sufficient to make the hydrogen peroxide evaporate. Within the plate vaporiser the supply of another fluid is envisaged, typically air, which acts as a support, i.e. it has the task of transporting the hydrogen peroxide vapours towards the surface to be sterilised. An example of this type of vaporiser is described in patent EP2455106. Another apparatus, known for example by document EP1135213, envisages the use of a nebuliser nozzle to atomise the hydrogen peroxide into an air flow, which again has a support function. The mixture of air and nebulised hydrogen peroxide passes through a heat exchanger so as to change the state of the hydrogen peroxide from liquid to vapour.

Finally, document WO98/34649 describes a vaporisation device split into two parts: the liquid steriliser is vaporised into the first part of the device and subsequently the sterilising vapours undergo a second heating, still within the same vaporisation device.

In all the known examples, the vaporisation apparatus must be arranged as near as possible to the zone in which the decontamination takes place, ideally close to the steriliser. In fact, it is fundamental for the hydrogen peroxide to completely evaporate and remain in the gaseous phase until it is time for sterilisation. The complete evaporation of the hydrogen peroxide allows the process to be kept under control over time so as to control the concentration of the steriliser on the surface to be treated.

In fact, in the process it is necessary to provide the correct quantity of liquid hydrogen peroxide to be vaporised per hour, which depends both on the desired concentration in the gaseous phase within the support fluid (connected with the required sterilisation performance and depending on the type of product filled) and on the flow rate of the support fluid (connected with how many containers need to be sterilised per hour, therefore with the size of the sterilising machine).

If at some points of the system, between the vaporisation and the sterilisation, the hydrogen peroxide vapours condensed, even in small quantities, the concentration value of the steriliser would be reduced and control of the process would be lost, reaching conditions in which the decontamination may not be effective.

In the process control, the temperature of the hydrogen peroxide vapours must be kept above the minimum value to guarantee sterilisation effectiveness. This aspect is particularly significant in the production of low-acid drinks, which require higher performance sterilisation for the packaging than other drinks.

A fundamental requirement in sterilising packaging intended to contain drinks is that the sterilising effect is greater than or equal to the value established and that it remains constant over time.

To obtain this condition it is important, as mentioned above, that condensate does not form and that the temperature of the vapours is above the threshold value. The two phenomena are related: by managing to keep the system above the dew point the formation of "cold" areas where the vapours could condense is less likely.

By keeping the vaporisation system as close as possible to the point in which the sterilisation takes place, the temperature of the vapours would be more easily kept above the minimum values and the probability of forming condensate would be reduced.

However, such positioning is not possible when the quantity per hour of liquid hydrogen peroxide to be vaporised becomes significant (e.g. over 6 kg/h) and therefore the dimensions of the vaporisation apparatus become such that they are not compatible with the available space on board or near the sterilising machine. In this case, the connection piping between the vaporiser and the steriliser, although insulated or even made in some sections with electrically heated pipes, is a heat dissipation element such that the temperature of the vapours at the sterilisation nozzles (i.e. in the point at which they come into contact with the packaging) may not exceed the minimum value for being able to guarantee the required sterilisation performance.

In these cases, it is useless or even negative to increase the temperature of the vaporisation apparatus. In fact, increasing the temperature of the vapours in contact with the packaging is not proportional to the increase in vaporisation temperature and increasing the vaporisation temperature generates in the vaporiser degradation of the hydrogen peroxide and calefaction, which reduce the vaporisation efficiency.

The situation becomes even more critical when the machine restarts, for example, after an interruption due to a prolonged stop of the line. In fact, after a prolonged stop, before starting the vaporisation of the hydrogen peroxide, the system must be brought to a higher temperature than the dew point to prevent the formation of condensate and to guarantee that the temperature of the vapours exceeds the minimum value. Therefore, a certain period of time is required, which represents downtime with no production.

Document EP 0956054 discloses a method for vaporising and superheating a sterilising agent for application to generic surfaces to be sterilized.

Document US 5173259 discloses a method for sterilising pipes that have been pre-heated in a heat-exchanger.

Document EP 0321908 discloses a method for sterilising a container according to the preamble of claim 1.

In this context, the technical task underpinning the present invention is to provide a method for sterilising containers made of polyethylene or high-density polyethylene, which obviates the drawbacks of the prior art as cited above.

In particular, an object of the present invention is to provide a method for sterilising containers made of polyethylene or high-density polyethylene that ensures the desired degree of sterility is obtained and maintained over time, without having to increase the structural complexity.

The stated technical task and specified objects are substantially achieved by a method for sterilising containers made of polyethylene or high-density polyethylene, comprising the steps of:
- generating an air flow;
- introducing a watery composition containing a sterilising liquid agent inside the air flow acting as a carrier and vaporising the mixture obtained thereof in a vaporisation device;
- sending the mixture to the containers to be sterilised, characterised in that it comprises a step of
   heating the mixture of air, water vapour and evaporated sterilising agent inside a post-heating unit that is structurally and functionally separate from said vaporisation device before sending it to the containers;
- retroactively regulating the power of the post-heating unit as a function of the temperature of the mixture exiting the post heating unit, said temperature being measured at the inlet of a machine for sterilising containers.

Preferably, the mixture is generated at a temperature of about 110°C and the post-heating unit is configured so as to increase the temperature of the mixture by about 20°C.

Additional features and advantages of the present invention will become more apparent from the approximate and thus non-limiting description of a preferred but not exclusive embodiment of a method for sterilising containers made of polyethylene or high-density polyethylene.

This description will be given below with reference to the attached drawings, provided solely for illustrative and therefore non-limiting purposes, in which:
- figure 1 illustrates a scheme of a first embodiment of an apparatus for sterilising a container, used in the method of the present invention;
- figure 2 illustrates a scheme of a second embodiment of an apparatus for sterilising a container, used the method of the present invention;
- figure 3 illustrates a schematic plan view of a machine for sterilising containers for use in the method of the present invention.

With reference to the figures, 1 indicates an apparatus for sterilising a container 100.

In particular, the container 100 is a parison or a bottle made of polyethylene (known as PET, which stands for polyethylene terephthalate) or high-density polyethylene (known by the acronym HDPE).

Alternatively, the container 100 is a pouch or flexible container.

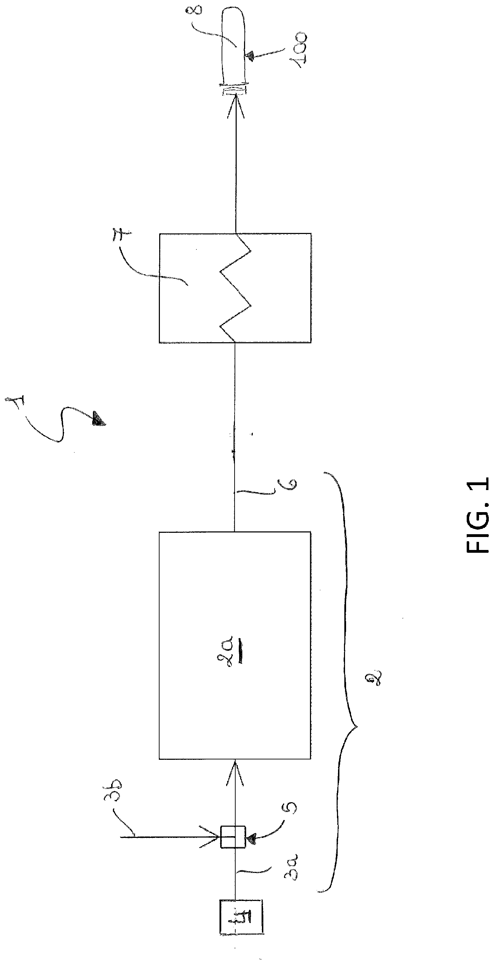

According to a first embodiment, for example, illustrated in figure 1, the sterilisation apparatus 1 comprises a vaporisation device 2 having a first inlet 3a for air and a second inlet 3b for supplying a watery composition containing a liquid sterilising agent. In particular, the liquid sterilising agent is hydrogen peroxide. Preferably, the concentration of hydrogen peroxide in the aqueous compound is about 35%.

Means are provided for generating an air flow 4 which communicate with the first inlet 3a of the vaporisation device 2.

For example, the means for generating an air flow 4 comprise a side-channel blower. Alternatively, for low flow rates, the means for generating an air flow 4 comprise a source of compressed air.

The vaporisation device 2 comprises a heat exchanger 2a, for example, an electrical or vapour heat exchanger of the plate or tube-in-tube type. The heat exchanger 2a is used to provide the necessary heat for changing the phase of the hydrogen peroxide from the liquid to the vapour state. Preferably, the air is filtered, for example, by making it pass through HEPA filters (not illustrated) before being sent to the heat exchanger 2a.

The sterilisation apparatus 1 comprises supply means 5 placed in communication with the second inlet 3b and configured to supply the watery composition containing the liquid sterilising agent into the vaporisation device 2, through the second inlet 3b.

Preferably, the supply means comprise a nebuliser nozzle 5 arranged along the first air inlet 3a. For example, the nebuliser nozzle 5 is a compressed air nozzle or a piezoelectric nozzle or is a pneumatic nozzle. The watery composition is sent to the nebuliser nozzle 5 through, for example, a pump or a tank pressurised with a gas.

The vaporisation device 2 further comprises an outlet 6 dispensing a mixture comprising air, water vapour and the evaporated sterilising agent. In fact, inside the heat exchanger 2a, the watery composition containing the sterilising agent evaporates and therefore generates the aforementioned mixture of air, water vapour and evaporated sterilising agent which exits from the vaporisation device 2 through the outlet 6.

The evaporated sterilising agent exiting from the heat exchanger 2a has the concentration envisaged for the sterilisation.

Originally, before being sent to the container 100 to be sterilised, the mixture thus obtained crosses a post-heating unit 7 which keeps its temperature above the dew point and therefore keeps it in the gaseous state. The post-heating unit 7 consists of a heat exchanger, for example, powered by electricity or vapour.

Such post-heating unit 7 is placed immediately upstream of a treatment chamber 8 of the container 100 to be sterilised.

In the embodiments described and illustrated herein, the treatment chamber 8 is the internal volume of the container (e.g. bottle) 100, i.e. the space delimited by the walls and the bottom of the container 100.

In an alternative embodiment (not illustrated), the treatment chamber 8 is a chamber containing the container 100, therefore it is the external walls of the bottle 100 that are treated, and potentially also the internal ones.

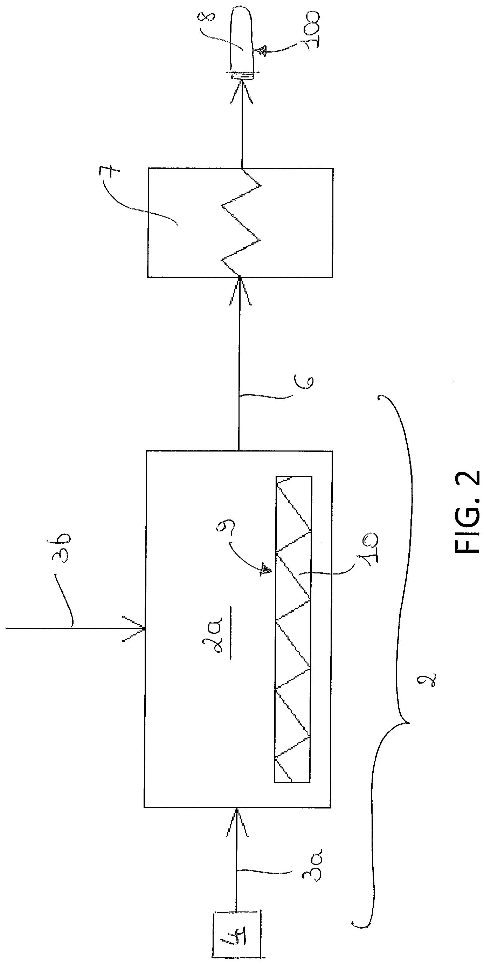

A second possible embodiment of the vaporisation apparatus 1, for example illustrated in figure 2, differs from the previous one as the vaporisation device 2 comprises a plate 9 associated with electrical resistances 10 so as to be heated. The first inlet 3a and the second inlet 3b are separate and the supply means 5 consist of a conduit configured to make the watery composition drip onto the plate 9. The hydrogen peroxide evaporates and the air is taken away (which acts as a carrier) out of the vaporisation device 2 through the outlet 6. The means for generating the air flow 4 are identical to those described for the first embodiment.

In a further variation (not illustrated), the supply means 5 consist of an ejector that draws the watery composition into the air flow in the vaporisation device 2.

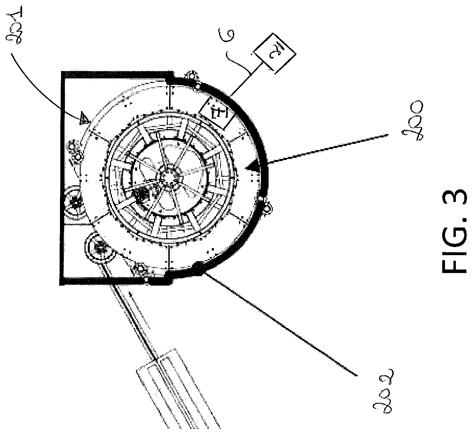

With reference to figure 3, 200 indicates a machine for sterilising containers 100, hereinafter indicated for short as "machine" 200.

The machine 200 comprises a rotary carousel 201 provided with a plurality of treatment stations for treating the containers 100. In each treatment station a dispensing nozzle (not illustrated) is arranged.

The machine 200 comprises an isolator 202 configured to separate the rotary carousel 201 from the external environment.

An example of a system comprising isolators suitable for application in the machine 200 is described and illustrated in document EP2279850 in the Applicant's name, incorporated herein for reference purposes.

The machine 200 further comprises a sterilisation apparatus 1 as previously described.

A rotary distributor, not illustrated, is arranged within the isolator 202 and is configured to receive from the sterilisation apparatus 1 and distribute to the dispensing nozzles of the rotary carousel 201 the mixture of air, water vapour and evaporated and heated sterilising agent. An example of a rotary distributor is described and illustrated in document WO2012/150513 in the Applicant's name, incorporated herein for reference purposes.

In particular, the vaporisation device 2 of the sterilisation apparatus 1 is arranged in a lateral zone of the rotary carousel 201 and the post-heating unit 7 is located in proximity of the rotary carousel 201.

In accordance with a first embodiment, the post-heating unit 7 is located on an upper external surface of the isolator 202, upstream of the rotary distributor.

Alternatively, the post-heating unit 7 is located inside the isolator 202, upstream of the rotary distributor.

In other words, the post-heating unit 7 is located in proximity to the machine 200 so as to be able to bring the temperature of the vapours to values higher than the minimum set for obtaining the required sterilisation performance.

The power of the post-heating unit 7 is retroactively regulated as a function of the temperature of the mixture exiting from the post-heating unit 7 measured at the inlet of a machine 200. By regulating and monitoring such temperature it is possible to have a temperature at the nozzles higher than the minimum temperature (e.g. 80°C).

The method for sterilising containers made of polyethylene or high-density polyethylene, according to the present invention, is described below.

In particular, such method comprises the steps of generating an air flow and introducing into the air flow a watery composition containing a sterilising liquid agent. Such watery composition carried by the air is vaporised so as to obtain a mixture of air, water vapour and evaporated sterilising agent. Originally, a heating step is envisaged (or better, "post-heating") of the mixture of air, water vapour and evaporated sterilising agent before it is sent to the containers 100.

In particular, the air is supplied into the vaporisation device 2 and the watery composition containing the sterilising liquid agent is introduced into the air flow. Such introduction preferably takes place by injection, for example, by nebulising the watery composition through the nebuliser nozzle 5. See, for example, the first embodiment illustrated in figure 1. Alternatively, the air is supplied into the vaporisation device 2 and the watery composition containing the sterilising liquid agent is made to drip onto the plate 9 (see figure 2).

In the heat exchanger 2a the watery composition containing the sterilising agent evaporates completely. Thus, the mixture of air, water vapour and evaporated sterilising agent is obtained.

The mixture thus obtained passes into the post-heating unit 7 placed immediately upstream of the treatment chamber 8. Advantageously, the post-heating unit 7 allows the mixture to be kept in the gaseous state compensating for losses suffered in the transport conduit of the mixture (not illustrated).

In particular, the vaporisation device 2 is configured to generate a mixture at a temperature of about 110°C and the post-heating unit 7 is configured to increase the temperature of the mixture by about 20°C.

The mixture remains within the post-heating unit 7 for a time interval of about 1 second.

The pressure of the air and/or the mixture is about 300 mbar. Subsequently, the mixture is drawn into the container 100, brushing against its inside walls. In particular, the inside walls of the container 100 are kept at a temperature less than or equal to the condensation temperature of the hydrogen peroxide vapour, i.e. less than 60°C.

In this way, the hydrogen peroxide condenses on the walls. Subsequently, it is evaporated by means of the delivery of hot air.

The concentration of sterilising agent in the mixture is determined by measuring the flow rate of the carrier and the flow rate of sterilising agent at the inlet to the vaporisation device 2, a concentration which is kept constant through a retro-activated control that operates on the dedicated valves so as to keep the flow rates within a pre-established range.

According to another variant, the VHP could even remain in the vapour phase (without condensing).

The characteristics of the method for sterilising containers made of polyethylene or high-density polyethylene, according to the present invention, are clear, as are the advantages.

In particular, by heating the gaseous mixture (formed by air, water vapour and hydrogen peroxide) prior to sending it to the container, the concentration of the sterilising agent is kept under control (preventing its condensation prior to the contact with the walls of the container), thus ensuring that the desired degree of sterility is obtained and maintained over time.

This result was obtained by inserting a post-heating unit structurally and functionally separate from the vaporisation device and operating on the mixture containing the completely vaporised sterilising agent in the vaporisation device.

The proposed solution therefore provides for the insertion of a second heat source placed downstream of the vaporisation device, in proximity to the sterilising machine, i.e. to the point in which the hydrogen peroxide vapours are used for the sterilisation.

In this way it is possible to increase the strength and reliability of the system over time.

The specific industrial application of this method is for the sterilisation of parison, which will then be blown in a sterile manner within a sterile blower. For this application it is necessary to place the vaporisation device far from the sterilising machine. The post-heating unit is placed in proximity to the sterilising machine, for example, on the roof of its isolator, just before the inlet to the isolator of the pipe which takes the hydrogen peroxide vapours to the rotary distributor. Installation outside the isolator is therefore simple and safe, but it is also possible to install the post-heating unit inside the isolator of the sterilising machine.

## Claims

1. Method for sterilising containers (100) comprising the steps of:
generating a flow of air;
introducing a watery composition containing a sterilising liquid agent inside the air flow and vaporising the mixture obtained thereof in a vaporisation device (2);
sending said mixture to the containers (100) to be sterilised,
**characterised in that** it comprises a step of heating the mixture of air, water vapour and evaporated sterilising agent inside a post-heating unit (7) that is structurally and functionally separate from said vaporisation device (2) before sending it to said containers (100);
retroactively regulating the power of the post-heating unit (7) as a function of the temperature of the mixture exiting the post heating unit (7), said temperature being measured at the inlet of a machine (200) for sterilising containers (100).

2. Method for sterilising containers (100) according to claim 1, wherein said mixture is generated at a temperature of 110°C and the post-heating unit (7) is configured so as to increase the temperature of the mixture by 20°C.

## Patentansprüche

1. Verfahren zur Behältnissterilisation (100), umfassend die Schritte von:
Erzeugen eines Luftstroms;
Einführen einer wässrigen Zusammensetzung, die ein sterilisierendes flüssiges Mittel im Luftstrom enthält, und Verdampfen der daraus erhaltenen Mischung in einer Verdampfungsvorrichtung (2);
Senden der Mischung an die zu sterilisierenden Behältnisse (100),
**dadurch gekennzeichnet, dass** es einen Schritt des Erhitzens der Mischung aus Luft, Wasserdampf und verdampftem Sterilisierungsmittel innerhalb einer Nachwärmeinheit (7) umfasst, die strukturell und funktionell von der Verdampfungsvorrichtung (2) getrennt ist, bevor sie zu den Behältnissen (100) gesendet wird;
nachträgliches Regeln der Leistung der Nachwärmeinheit (7) in Abhängigkeit von der Temperatur der aus der Nachwärmeinheit (7) austretenden Mischung, wobei die Temperatur am Einlauf einer Maschine (200) zum Sterilisieren von Behältnissen (100) gemessen wird.

2. Verfahren zur Behältnissterilisation (100) nach Anspruch 1, wobei die Mischung bei einer Temperatur von 110 °C erzeugt wird und die Nachwärmeinheit (7) so konfiguriert ist, dass sie die Temperatur der Mischung um 20 °C erhöht.

## Revendications

1. Procédé pour stériliser des récipients (100), comprenant les étapes de :
générer un flux d'air ;
introduire une composition aqueuse contenant un agent liquide stérilisant à l'intérieur du flux d'air et vaporiser le mélange obtenu de celle-ci dans un dispositif de vaporisation (2) ;
envoyer ledit mélange aux récipients (100) à stériliser,
**caractérisé en ce qu'**il comprend une étape consistant à chauffer le mélange d'air, la vapeur d'eau et l'agent stérilisant évaporé à l'intérieur d'une unité de post-chauffage (7) étant structurellement et fonctionnellement séparée dudit dispositif de vaporisation (2) avant de l'envoyer auxdits récipients (100) ;
régler rétroactivement la puissance de l'unité de post-chauffage (7) en fonction de la température du mélange sortant de l'unité de post-chauffage (7), ladite température étant mesurée à l'entrée d'une machine (200) pour stériliser des récipients (100).

2. Procédé pour stériliser des récipients (100) selon la revendication 1, dans lequel ledit mélange est généré à une température de 110 °C et l'unité de post-chauffage (7) est configurée de sorte à augmenter la température du mélange de 20 °C.
